Europäisches Patentamt

European Patent Office

Office européen des brevets

(19)

(11) EP 0 926 484 A2

(12) **EUROPEAN PATENT APPLICATION**

(43) Date of publication:
30.06.1999 Bulletin 1999/26

(51) Int Cl.⁶: **G01N 21/86**

(21) Application number: 98310464.7

(22) Date of filing: 18.12.1998

(84) Designated Contracting States:
**AT BE CH CY DE DK ES FI FR GB GR IE IT LI LU MC NL PT SE**
Designated Extension States:
**AL LT LV MK RO SI**

(30) Priority: 24.12.1997 JP 36633797

(71) Applicant: **TERUMO KABUSHIKI KAISHA Tokyo 151-0072 (JP)**

(72) Inventors:
• **Morikawa, Naoki**
  **Nakakoma-gun, Yamanashi (JP)**
• **OOmori, Tooru**
  **Nakakoma-gun, Yamanashi (JP)**
• **Uchida, Takao**
  **Nakakoma-gun, Yamanashi (JP)**
• **Yoshida, Kazuhito**
  **Higashiyatsushiro-gun, Yamanashi (JP)**
• **Kasai, Masaaki**
  **Nakakoma-gun, Yamanashi (JP)**

(74) Representative:
**Harrison, David Christopher et al MEWBURN ELLIS York House 23 Kingsway London WC2B 6HP (GB)**

(54) **Test paper and analyte collecting head**

(57) An analyte measuring tip 5 is provided with a cap 51, a tube 52 projecting from an end wall 511 of the cap 51, and a test paper 53 set in place on the inner side of the end wall. The test paper 53 is the product of superposition of a first layer 53a and a second layer 53b. The first layer 53a is formed of a porous membrane carrying thereon a reagent capable of reacting with a specific component (such as, for example glucose) in blood and proportionately assuming a color and the second layer 53b is formed of a porous membrane fulfilling the function by separating red blood cells from the blood through filtration while allowing the colored vehicle to reach its further face, where it may be examined by an automated test device. The test paper per se is also inventive.

Fig. 4

## Description

This invention relates to a test paper and an analyte measuring tip for measuring the amount of a target analyte in a given sample as, for example, in the measurement of blood sugar level in a blood sample.

Blood sugar measuring devices which work by optically measuring the degree of coloration of a test paper (colorimetry) which assumes a color proportionately to the amount of glucose or other analyte in blood, are known.

In a conventional blood sugar measuring device of this construction, the measurement of the color of the test paper has been carried out in a light measuring part provided with a light-emitting element and a light-receiving element by projecting light on the test paper and measuring the intensity of the light reflected from the test paper.

This device, subsequently to the work of supplying and spreading a blood sample on the test paper, requires the test paper to be inserted into a space where it can be shielded from ambient light before the measurement of the blood sugar level can be started. Besides suffering from this inconvenient mode of operation, this device has the problem that the interval between the time the blood is supplied and the time the measurement is started is not fixed, which gives rise to errors.

Thus, there is a need for a colorimetric analyser for analytes such as blood sugar, which is capable of continuously and automatically performing a series of operations from supply and development of samples on the test paper to measurement.

The conventional test paper for prior art devices comprises a sheet substrate formed of a porous material capable of absorbing a sample, and a reagent deposited on the substrate. This test paper has pores in the sheet substrate with diameters of the order of 0.5 μm, and is at a disadvantage since it can be insufficiently permeable or attractive to water and can require an unduly long time for a sample to be fully measurable. The fact that the time for developing a sample is long as described above is unfavorable particularly for an automatic blood sugar measuring device.

The object of this invention is to provide a test paper and an analyte measuring tip which permit reduction in the time required for developing a sample and allow measurements to be performed with high accuracy.

The test paper and the analyte measuring tip according to this invention will now be described below with reference to preferred embodiments which are illustrated in the accompanying drawings.

Fig. 1 is a side sectional view illustrating the internal structure of a device for measuring an analyte in blood to be used with an analyte measuring tip embodying this invention loaded therein;

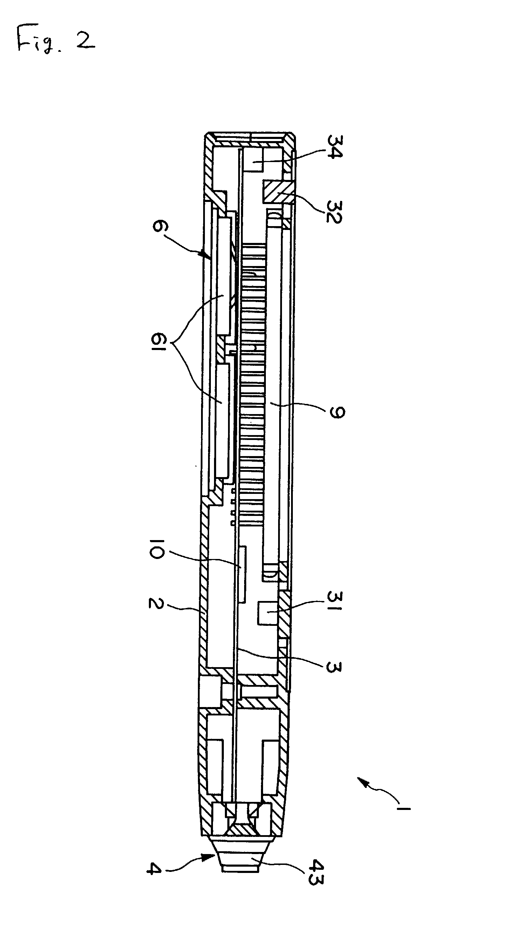

Fig. 2 is a plan sectional view of the analyte measuring device;

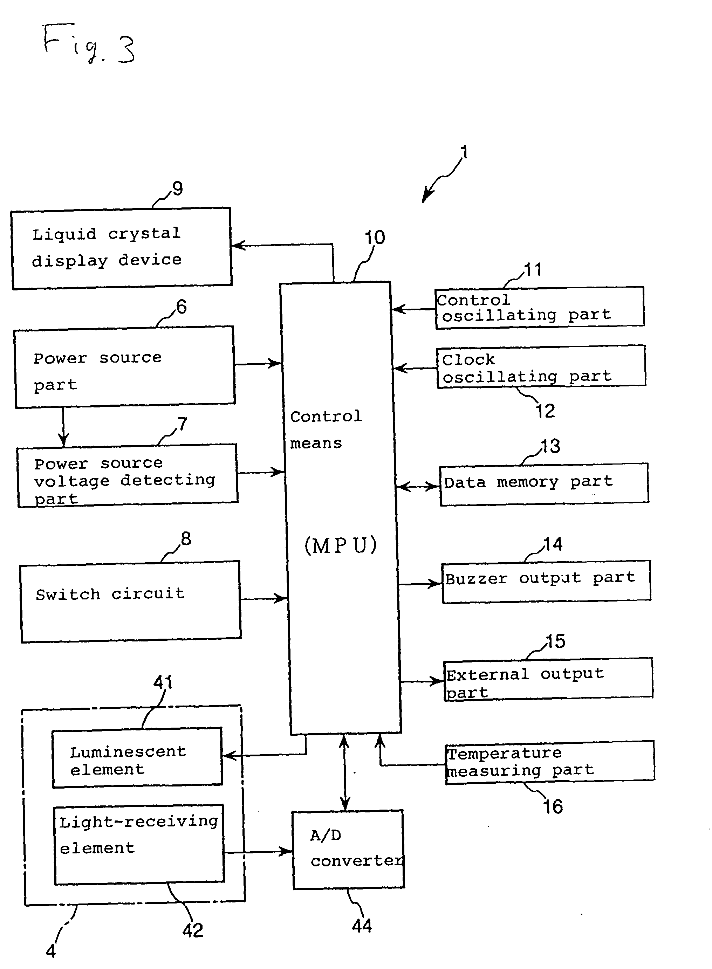

Fig. 3 is a block diagram illustrating the circuit configuration of the analyte measuring device;
Fig. 4 is a longitudinal section illustrating a typical construction of the analyte measuring tip;
Figs. 5 and 6 are perspective views illustrating the constructions respectively of the sample inlet side and the sample outlet side terminal part of a delivery tube of the tip;
Figs. 7 and 8 are diagrams showing possible dimensions of various parts;
Fig. 9 is a longitudinal section illustrating the tip in a state in which it is loaded in the analyte measuring device;
Figs. 10 and 11 are perspective and plan views illustrating a typical construction of the test paper of this invention;
Fig. 12 is a cross section taken through Fig. 11 along the line A-A; and
Fig. 13 is a side view illustrating the analyte measuring tip in a state in which the tip is used for collecting a sample.

As illustrated in these drawings, an analyte measuring device 1 for a blood sample is provided with a casing 2 with a printed circuit 3 disposed therein. Further, the casing 2 is provided in one end portion thereof with a light measuring part 4. The casing 2 has a window with a liquid crystal display device (LCD) 9.

On the printed circuit 3 is installed a microcomputer 10 as control means operating to control various functions of the analyte measuring device 1. This control means 10 has built therein an arithmetic part for computing a target analyte in blood (such as, for example, glucose) based on signals from the light measuring part 4. This arithmetic part, as occasion demands, performs such correcting treatments as, for example, computation of hematocrit correction and computation of temperature correction.

The light measuring part 4 is provided with a luminescent element (light-emitting diode) 41 and a light-receiving element (photodiode) 42. These elements are accommodated and retained in a holder 43. The luminescent element 41 is electrically connected to the control means 10 and the light-receiving element 42 is electrically connected to the control means 20 via an amplifier (not shown) and an analogue/digital converter 44.

The luminescent element 41 is actuated by a signal from the control means 10 and caused to emit a pulse of light at prescribed intervals. The light pulse cycle is approximately in the range of 0.5 - 3.0 m.sec. and the duration of one pulse is approximately in the range of 0.05 - 0.3 m.sec. The wavelength of the light of this pulse is, for example, approximately in the range of 500 - 720 nm.

On the holder 43 which is the part onto which a test paper is to be loaded, an analyte measuring tip (hereinafter referred to simply as "tip") 5 having a test paper 53 therein is loaded in such a way as to be freely detachable. At this stage, a general description of the tip 5 will be given. Preferred constructions of the tip 5 and of the test paper 53 will be described in detail later.

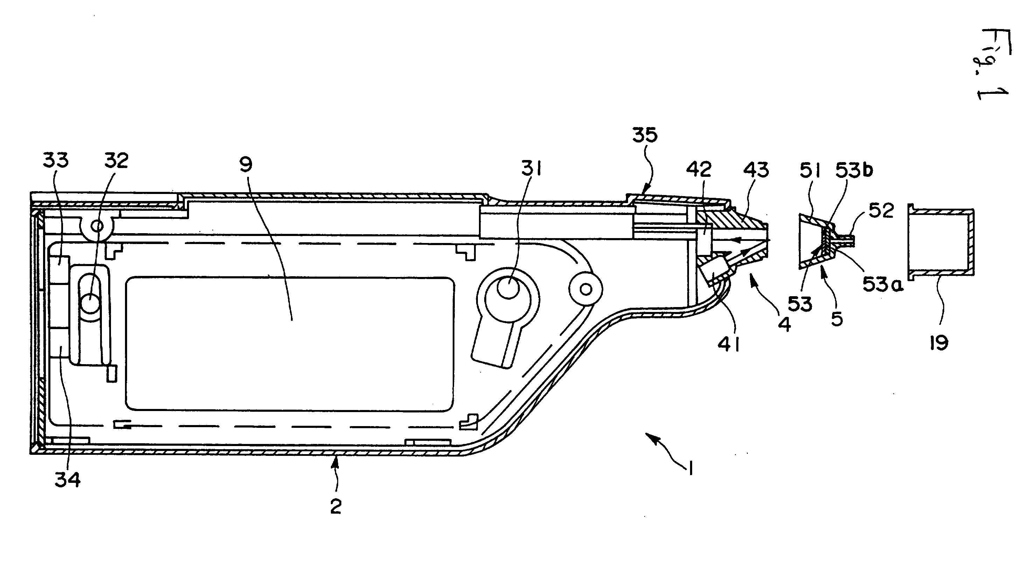

The tip 5 is composed of a transparent or translucent tapering cylindrical cap 51. It may have colored transparency such as, for example, blue transparency. The test paper 53 set in place on the inner side of the bottom of the cap 51 (Fig. 1 refers). The test paper 53 is obtained by superposing a first layer 53a and opaque second layer 53b.

A tube 52 projects from the outer side of the bottom of the cap 51. When a sample such as blood is brought into contact with the leading end of this tube 52, the sample is aspirated into the tube 52 and transported to the test paper 53 by capillarity through a narrow conduit in the tube. The sample supplied to the approximately central part of the test paper 53 spreads in a radial pattern on the test paper 53 and is allowed to react with the reagent carried thereon and assume a color.

When the luminescent element 41 is lit while the tip 5 is loaded in the holder 43, the light emitted from the luminescent element impinges on the side of the test paper 53 which is the second layer 53b and produces reflected light. The intensity of the reflected light is equivalent to the intensity of coloration of the second layer 53b, namely to the amount of concentration of the target analyte in the sample. This reflected light is received by the light-receiving element 42 and subjected to photoelectric conversion therein. The light-receiving element 42 emits an analog signal corresponding to the amount of light received. The analog signal is amplified, when necessary, converted into a digital signal by the A/D converter 44, injected into the control means 10, and then stored in the first memory of a data memory 13.

The analyte measuring device 1 is further provided with a power source portion 6, a power source voltage detector 7, a switch circuit 8, a control oscillating portion 11, a clock oscillator 12, the data memory 13, a buzzer output 14, an external output 15, and a temperature measurer 16.

The power source portion 6 is loaded with a dry cell 61. The power source voltage detector 7 detects the voltage of the dry cell 61 and issues the result to the control means 10. Thus, the remaining power of the dry cell 61 can be checked.

The switch circuit 8 detects the input to a switch and injects the signal corresponding to the input into the control means 10. As concrete examples of this switch, a power source switch, memory data read switch, clock setting/changing switch, resetting switch, buzzer ON/OFF selecting switch, and 50 Hz/60 Hz commercial power source frequency selecting switch may be cited.

The power source switch can be turned ON/OFF by depressing an operating button 31.

The other switches can be actuated by manipulating the operating button 31, and operating members 32, 33, 34, etc., either alone or in combination.

The control oscillating portion 11 is a timer. It oscillates clock pulses at fixed intervals and supplies operating standard signals for a microcomputer (microprocessing unit: MPU) of the control means 10.

The clock oscillating part 12 is a clock for specifying an absolute time (date and time). It oscillates clock pulses at fixed intervals and supplies an operating standard signal for the clock control circuit built in the control means 10.

The data memory 13 is provided with a first memory (RAM), a second memory (ROM), and a third memory (nonvolatile RAM). The size of the result of light measurement input from the light measuring part 4 is stored in the first memory in accordance with a prescribed format.

In the second memory, the relation (calibration curve), tabulated in advance, between the absorbance computed from the magnitude of the result of the measurement of light and the amount of a target analyte in blood (hereinafter represented by "blood sugar level") is stored.

In the third memory, a proofreading value peculiar to a relevant device is stored in advance. The term "proofreading value" as used herein embraces specific magnitudes of the amounts of reflected light and correction coefficients for the computation of final absorbance, for example.

The blood sugar level is computed on the basis of such sets of data stored in the first second and third memories as described above. Preferably, in this case, the blood sugar level is computed based on the ratio of the intensity of the reflected light from the test paper 53 after the development of color to the intensity of the reflected light prior to the development of color.

The buzzer output 14 actuates a buzzer and causes emission of sound based on the signal from the control means 10.

The external output 15 is intended to issue the data such as blood sugar level obtained by computation to a device such as, for example, a personal computer. In this case, the external output 15 has such a communication driver as, for example, RS232C, built therein. Where infrared communication is to be carried out, the external output 15 has an infrared ray-emitting element and a drive circuit therefor built therein.

The temperature measurer 16 is provided with a temperature sensor such as a thermistor capable of measuring the ambient temperature. The temperature measurer 16 performs measurement of temperature from time to time. The temperature data so obtained is stored in the first memory of the data memory 13. The temperature data read from the first memory is injected into the control means 10 and utilized therein for correcting and computing the correct compensation for the blood sugar level in relation to temperature.

Preferred embodiments of the test paper of this invention and of the analyte measuring tip furnished therewith will be described below.

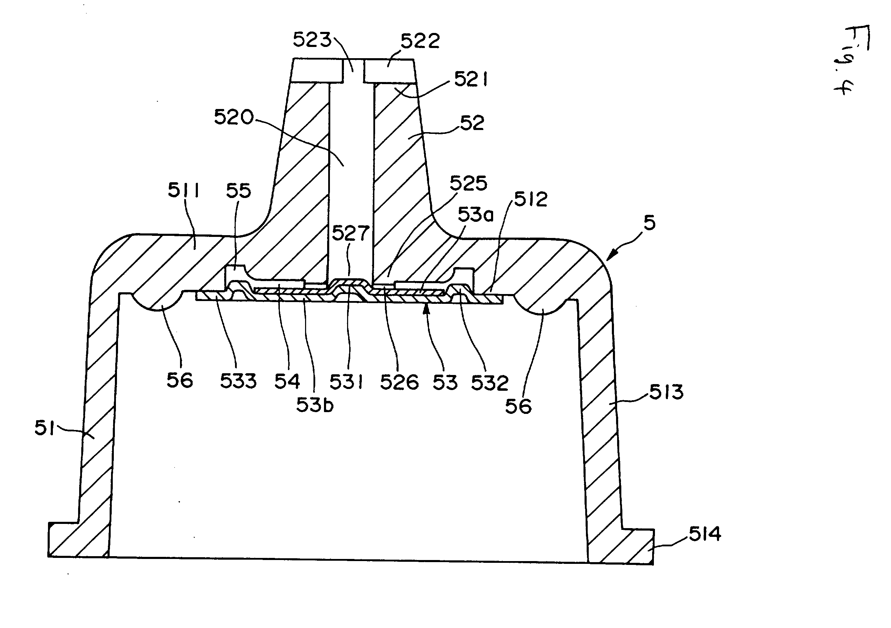

The lower side of the diagram of Fig. 4 will be called the "basal terminal" and the upper side as the "leading terminal" in the following description.

The tip 5, as illustrated in Fig. 4, is composed of the cap 51, the tube 52 projecting from an end wall 511 of the cap 51, and the test paper 53 set in place inside the cap 51.

The cap 51 is intended to support the test paper 53 and, at the same time, act to allow the cap 5 to be put in place on the light measuring part 4 of the analyte measuring device 1.

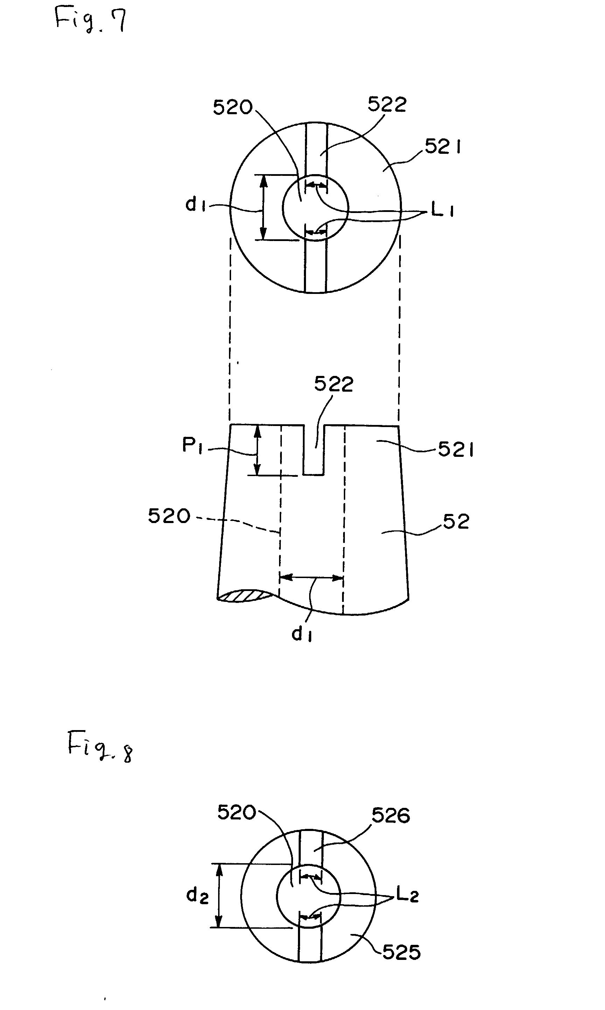

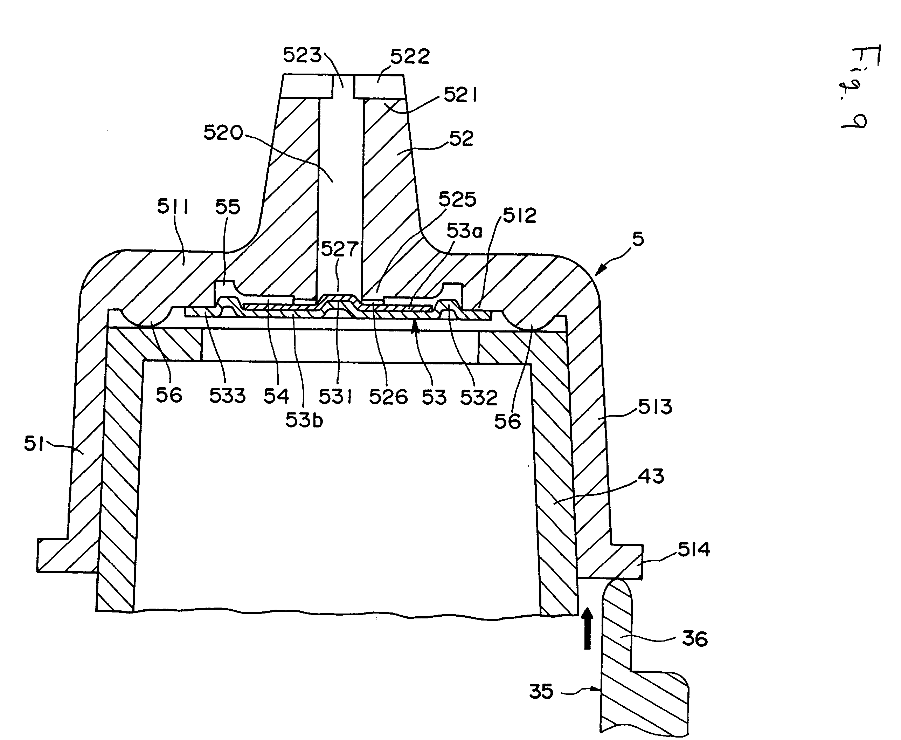

Since a used cap can be removed from the measuring device by means of a release button 35 without hand-contact as illustrated in Fig. 1 and Fig. 9, the possibility of contamination by the blood is reduced. If the tip is kept covered with a cap 19 while it is being removed from or put onto the measuring device, contamination from the handler is very unlikely.

The cap 51 is composed of the end wall 511, a barrel part 513, and a flange 514 formed on the outer periphery of the basal terminal of the barrel part 513. Further, inside the end wall 511, a pedestal 512 for fixing the test paper 53 is formed. The test paper 53 is fixed by its outer peripheral part 533 to the pedestal 512 by e.g., fusion or adhesion.

As illustrated in Fig. 9, the tip 5 is loaded in the light measuring part 4 of the analyte measuring device for blood by inserting holder 43 of the light measuring part 4 inside the barrel part 513 of the cap 51.

The barrel part 513 is preferably tapered so that the inside diameter thereof converges gradually toward the end wall as illustrated in Fig. 4 and Fig. 9. By this taper, the tip can be infallibly inserted and loaded even when the inside diameter of the barrel differs slight from the outside diameter of the holder 43.

The flange 514 functions as a grip part for allowing fingers to exert necessary force in attaching or detaching the tip 5 from the light measuring part 4 of the analyte measuring device 1.

Though the present embodiment, as depicted above, has the end wall 511, barrel part 513, flange 514 and tube 52 wholly formed integrally, they could be formed as separate members and eventually joined.

The tube 52 is intended to collect the sample of blood. It has a sample inlet conduit 520 running in a direction nearly perpendicular to the general plane of the test paper 53 and has a sample inlet 523 formed at the leading terminal thereof and a sample outlet 527 formed at the basal terminal thereof.

Since the blood is supplied through the sample inlet conduit 520 to the test paper 53 by capillarity, the inner diameter or average diameter of the sample inlet conduit 520 is properly approximately in the range of 0.2 - 2.0 mm, preferably 0.3 - 1.0 mm. If the inner diameter of the sample inlet conduit 520 is unduly large, the transfer of the blood by capillarity will become difficult. If the inner diameter is unduly small, the blood will be supplied slowly and the time required for supplying the blood in an ample amount to the test paper 53 will be increased.

The inside diameter (cross-sectional area) of the sample inlet conduit 520 may be constant or be varied along its longitudinal direction.

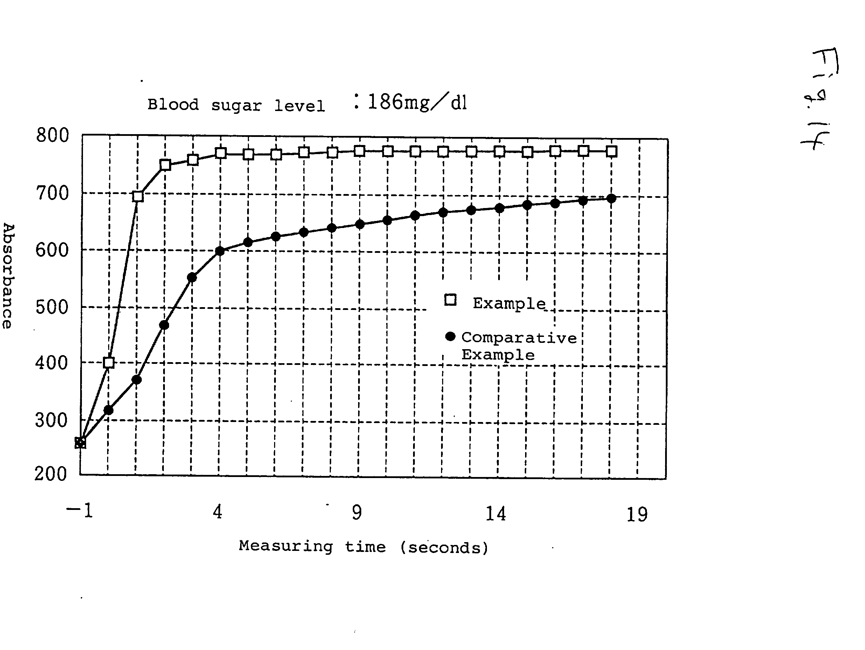

The total length of the sample inlet conduit 520 is preferably in the approximate range of 1 - 10 mm, more preferably in the approximate range of 2 - 5 mm. If the sample inlet conduit 520 is unduly long, transfer of the blood by capillarity takes an unduly long time, If the conduit is unduly short, blood 18 (Fig. 13) may contact and possibly adhere to the outer side of the end wall of the cap 51.

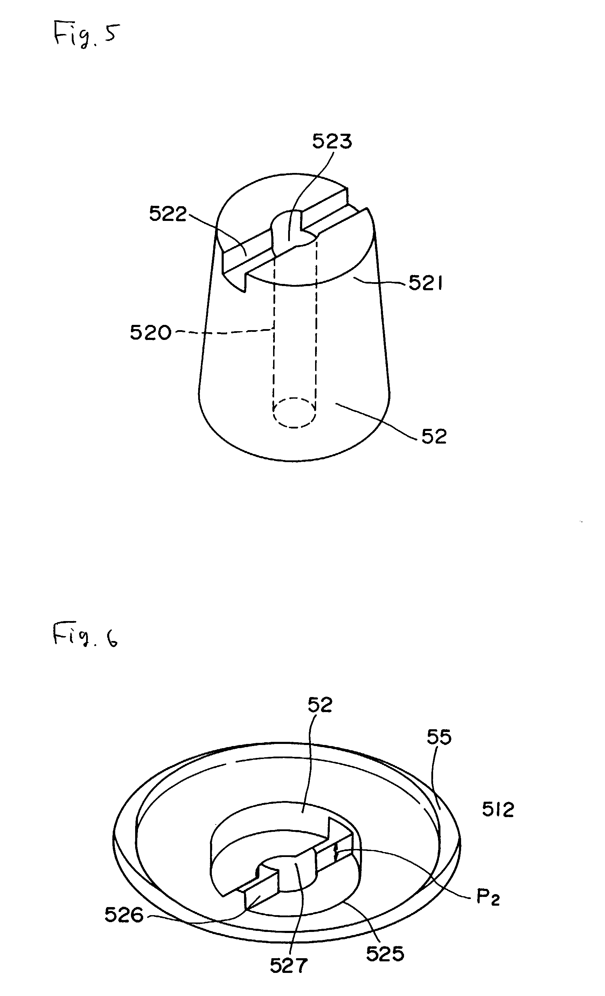

The leading terminal part and the basal terminal part of the tube 52 are respectively a sample inlet side terminal part 521 and a sample outlet side terminal part 525, as illustrated in Figs. 4 - 6.

On the terminal face of the sample inlet side terminal part 521, a groove 522 communicating with the sample inlet conduit 520 is formed. In the illustrated example, the groove 522 is a straight groove extending diametrically of the tube 52. The opposite ends of this groove 522 open in the outer peripheral surface of the tube 52.

The groove 522 secures a flow path for blood and allows supply of the blood to the test paper 53 to be effected smoothly and infallibly because the sample inlet conduit 520 is not closed when the terminal face of the sample inlet side terminal part 521 is brought into contact with a surface, such as that of a finger, during the collection of blood.

Referring to Fig. 7 if $L_1$ denotes the total peripheral length of the part forming the boundary between the groove 522 and the sample inlet conduit 520 and $d_1$ denotes the inside diameter of the sample inlet conduit 520 (the inner diameter near the sample inlet mouth 523), the relationship

$$L_1 \leq \Pi d_1 \times 50\% \qquad (I)$$

is preferred.

As a result, the start of the aspiration of blood by the sample inlet conduit 520 can be attained quickly and smoothly.

The depth P1 of the groove 522 is not particularly critical but may be selected, depending on the condition of the skin. Generally, it is preferably not less than 0.1 mm and more preferably in the approximate range of 0.2 -1.8 mm. If the groove 522 is unduly shallow, the passage of blood in it will become insufficient, especially if a high pressure is exerted on the skin.

The purpose of the groove 522 is that part of the terminal face of the sample inlet side terminal part 521 may not contact the skin when the terminal face is placed against the skin, and this may be achieved in different ways, for example by forming a plurality of grooves 522 radially (in the shape of a cross, for example) around the sample inlet mouth 523 of the sample inlet conduit 520 as the center or by forming them parallelly in such a manner as to touch the sample inlet conduit 520.

The sample outflow side terminal part 525 (Fig. 6) of the tube 52 (on the test paper 53 side) projects slightly toward the tip interior side (basal terminal side) from the end wall 511. On the terminal face of the sample inlet side terminal part 525, a second groove 526 communicating with the sample inlet conduit 520 is formed. In the illustrated example, the groove 526 is a straight groove extending in the direction of the diameter of the tube 52. The opposite terminals of this groove 526 open in the outer peripheral surface of the projecting part 525 of the tube 52.

Owing to the provision of the groove 526, the blood is spread quickly and uniformly over the test paper 53 and the measurement is obtained more accurately because the blood which has passed the sample inlet conduit 520 is released from the sample outlet mouth 527 and spread toward the outer periphery of the terminal part 525 via the groove 526 and then supplied to and developed on the test paper 53.

Referring to Fig. 8, if $L_2$ denotes the total peripheral length of the part forming the boundary between the groove 526 and the sample inlet conduit 520 and $d_2$ denotes the inside diameter of the sample inlet conduit 520 (the inner diameter near the sample outlet mouth 527), the following formula (II)

$$L_2 \leq \Pi d_2 \times 50\% \qquad (II)$$

is preferred.

As a result, the blood which has flowed out of the sample outlet mouth 527 of the sample inlet conduit 520 can be diffused and developed more quickly and smoothly toward the outer periphery of the test paper.

The depth P2 of the groove 526, though not particularly critical is preferred to be not less than 0.01 mm generally, and to be in the approximate range of 0.05 - 0.5 mm advantageously. If the depth P2 is unduly shallow, the groove 526 will possibly fail to fulfill its function properly.

As with groove 522, a plurality of grooves 526 may be formed radially (in the shape of a cross, for example) around the sample outlet mouth 527 of the sample inlet conduit 520 as the center or they may be formed parallelly in such a manner as to touch the sample inlet conduit 520.

Then, as illustrated in Fig. 4, a gap 54 is formed in the tube 52 side of the test paper 53, namely between the test paper 53 and the inner side of the end wall 511 of the cap 51. This gap 54 functions to aid in the distribution of blood on the test paper 53. To be specific, since the blood which has flowed out of the sample outlet mouth 527 of the sample inlet conduit 520 expands radially through the gap 54 by capillarity, distribution of blood on the test paper 53 (particularly on the first layer 53a) can be attained quickly and uniformly.

The depth of the gap 54, though not particularly critical, is preferred to be not less than 0.02 mm (average), particularly in the approximate range of 0.04 mm - 0.4 mm. A gap 54 having this depth can fulfill the function mentioned above very effectively. The depth of the gap 54 may be constant or may be varied (gradually decreased, for example) from the central part of the test paper 53 to the outer periphery thereof.

On the outer periphery of the gap 54, there is a sample reservoir 55 in the shape of an annular depression communicating with the gap 54 and of greater depth than the gap 54. As a result, the blood which has spread radially through the gap 4 is collected in the sample reservoir 55 and is obstructed from moving further towards the outer periphery, namely towards the position at which the test paper 53 is fixed by adhesion or fusion. Leakage of surplus blood, therefore, is prevented even when an excess of the blood is excessively supplied. As a result, blood contamination of the light measuring part 4 or other parts of the analyte measuring device 1 can be prevented.

Outside the pedestal part 512 on the inner face of the end wall 511, a spacer 56 acts as separating means to ensure absence of contact between the test paper 53 and the holder 43 when the tip 5 is loaded in the light measuring part 4 of the analyte measuring device 1.

This spacer 56, as illustrated in Fig. 9, is formed of a plurality of convex parts, here, four circumferentially spaced at angular intervals of 90°, laid out along the peripheral direction on the inner side of the end wall 511. During the loading of the tip 5 in the light measuring part 4, it lodges against the leading end of the holder 43 of the light measuring part 4.

Further the spacer 56, during the loading of the tip 5 in the light measuring part 4, also maintains fixed distances between the test paper 53 on the one part and the luminescent element 42 and the light-receiving element 42 of the light measuring part 4 on the other part. The spacer thus reduces errors of measurement due to variation in the distances mentioned above with consequent variation of the light values and contributes to accuracy of measurement.

The cap 51 and the tube 52 constructed as described above are formed of a material having a desired rigidity. As concrete examples of this rigid material, various resinous materials including acryl type resin, polystyrene, polyethylene, polypropylene, hard polyvinyl chloride, polycarbonate, polymethyl methacrylate, ABS resin, polyesters, polyphenylene sulfide (PPS), polyamide, polyimide, and polyacetal and polymer alloys and polymer blends containing at least one of the compounds mentioned above may be cited. Among other rigid materials enumerated above, such materials as acryl type resin which have high hydrophilicity and such materials which have undergone a treatment intended to impart hydrophilicity prove particularly advantageous because they are suitable for quick introduction and development of a sample.

Besides such physical activating treatments as, for example, plasma treatment, glow discharge, corona discharge, and irradiation with ultraviolet light, the impartation of hydrophilicity can be attained by the addition or application of surfactant, water-soluble silicon, hydroxypropyl cellulose, polyethylene glycol or polypropylene glycol.

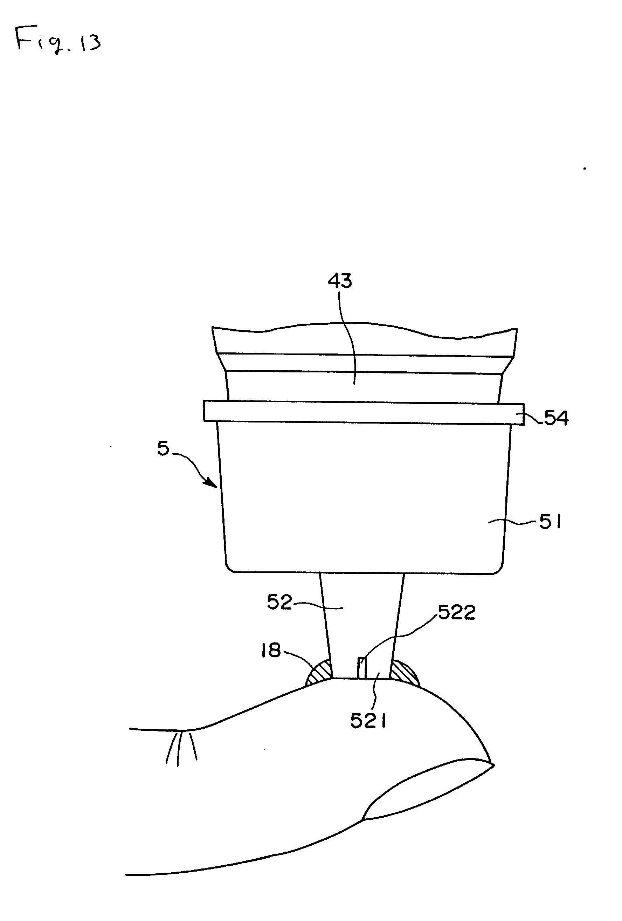

The shape and construction of the test paper 53 will be described with reference to Fig. 9 to Fig. 12.

Though the test paper 53 is preferred to have such a circular overall shape as illustrated, it is not necessarily so. For example, elliptic, square, rectangular, rhombic and other quadrilaterals, triangular, hexagonal, and octagonal shapes are possible.

When the test paper 53 has a circular shape, the outside diameter of the test paper 53 is preferably in the approximate range of 2 - 10 mm, more preferably in the approximate range of 3 - 6 mm.

The thickness of the test paper 53 is preferably in the approximate range of 0.02 - 1.0 mm, more preferably in the approximate range of 0.05 - 0.4 mm.

The test paper 53 is provided at the central part thereof, namely at the position fronting the sample inlet conduit 520, with a convex part 531 protruding toward the sample inlet conduit 520. Though the height of this convex part 531 is not particularly critical, at least the leading end of the convex part 531 is preferred to be insertable into the sample outlet mouth 527 of the conduit 520.

Therefore, the convex part 531 preferably has at least partly a diameter equal to or smaller than the inside diameter of the sample outlet mouth 527 of the sample inlet conduit 520 and also has a circular contour. The height of the convex part 531 is preferably in the approximate range of 0.02 - 1.0 mm, more preferably in the approximate range of 0.05 - 0.4 mm. The shape, size, etc. of the convex part 531 do not need to be limited to those specified above but may be properly selected to suit such factors as the cross section, shape, etc. of the sample inlet conduit 520.

The convex part 531 provided in the manner described above enables the blood which has passed through the sample inlet conduit 520 to be supplied more quickly to the test paper 53.

The test paper 53 is further provided radially inside its outermost periphery with an annular convex part 532 protruding in the same direction as and centered on the convex part 531. Its leading end is opposite to and may be inserted in the sample reservoir 55 mentioned above.

This annular convex part 532 has the function of regulating the development of blood on the test paper 53. It obstructs the outflow of excess blood radially beyond the periphery of the test paper 53b.

The diameter of the annular convex part 532, though not limited particularly, is preferably in the approximate range of 70 - 95%, more preferably in the approximate range of 85 - 95%, of the outside diameter of the test paper 53 (which is the diameter of its second layer 53b).

The width of the annular convex part 532 is preferably in the approximate range of 0.03 - 1.0 mm, more preferably in the approximate range of 0.05 - 0.5 mm. The height of the annular convex part 532 is preferably in the approximate range of 0.02 -1.0 mm, more preferably in the approximate range of 0.05 - 0.4 mm however.

The shape, dimensions (diameter, width, height, etc) of the annular convex part 532 may be freely selected to suit the shape and other features of the cap 51.

The convex part 531 and the annular convex part 532 which are constructed as described above may be formed, for example, by patterning in a die (projecting the basal terminal face of the test paper 53 by exertion of pressure as with a punch) or by mechanical cutting.

The test paper 53 constructed as described above is composed of the first layer 53a and the second layer 53b.

The first layer 53a is what results from depositing a reagent such as a coloring reagent on a carrier capable of absorbing a sample. Preferably, this carrier is formed of a porous membrane or other sheetlike porous substrate.

Particularly when the reagent used for the impregnation is a reagent system which undergoes a reaction such as an oxidase reaction using the oxygen in the air as a substrate, the use of a porous membrane as the carrier ensures sufficient supply of the oxygen from the air for the sample which has been developed on the first layer 53a and enables the reaction to proceed rapidly and therefore allows detection of the state of coloration without requiring removal of the sample or the filtrate thereof (red blood cells, etc.).

Examples of porous membranes for the first layer 53a are a non-woven fabric, woven fabric, stretched sheet, membrane filter, or filter paper.

As specific examples of the material for forming the porous membrane of the first layer 53a, polyesters, polyamides, polyolefins, polysulfones, celluloses, silicates, and fluorine type resins may be cited. The materials usable for the porous membrane include polyethylene terephthalate, polybutylene terephthalate, polyether sulfone, nitrocellulose, cellulose, glass, polytetrafluoroethylene (Teflon), nitrolized polyether sulfone.

The material for forming the porous membrane preferably is a material inherently possessing hydrophilicity or a material which has undergone a treatment adapted for imparting hydrophilicity because the layer is produced by the impregnation with an aqueous solution having a reagent dissolved therein and also because the layer is expected to absorb and develop a sample quickly. The treatment for the impartation of hydrophilicity is the same as described above.

As specific examples of the reagent for impregnating the porous membrane of the first layer 53a which is used for the measurement of blood sugar level, glucose oxidase (GOD) and peroxidase (POD) and such coloring agents as, for example, 4-aminoantipyrine and N-ethyl-N-(2-hydroxy -3-sulfo-propyl)-m-toluidine may be cited. Depending on the kind of analyte to be measured such reagents as, for example, ascorbic acid oxidase, alcohol oxidase, and cholesterol oxidase which react with the components of blood and the same coloring agents as mentioned above may be cited. Optionally, the reagent may incorporate therein a buffer solution such as a phosphoric acid buffer solution. Of course, the kind and the composition of the reagent are not limited to those which are mentioned above provided they will provide a coloration signal at the observed face of the second layer 53b.

The porous membrane of the first layer 53a of the kind mentioned above preferably contains pores of a diameter such that the blood cells, particularly the red blood cells, contained in a sample blood may be passed through the membrane. To be specific, the diameter of the pores in the first layer 53a preferably is in the approximate range of 8 - 50 µm, more preferably in the approximate range of 10 - 30 µm. If the pore diameter is unduly small, the amount of red blood cells to be passed will decrease and the developability of blood will be degraded. If the pore diameter is unduly large, the deposition of the reagent will be attained only with difficulty and the sensitivity of coloration will be degraded because coloration will be attained only with difficulty.

The first layer 53a described above has an outside dimension smaller than the outer dimension of the second layer 53b and it is positioned within the radially inner side of the annular convex part 532. Since the annular convex part 532 obstructs the outflow of the blood toward the outer peripheral side as described above, the first layer 53a which is responsible for the development of blood has need to be disposed radially within the annular convex part 532 but they could have equal outside diameters.

The thickness of the first layer 53a, though not particularly critical, is preferably in the approximate range of 10 - 1000 µm, more preferably in the approximate range of 50 - 200 µm.

The second layer 53b is formed of the same porous membrane (sheetlike porous substrate) as mentioned above.

As specific examples of the porous membrane, non-woven fabric, woven fabric, stretched sheet, membrane filter, and filter paper may be cited. The materials which are available for the formation of the porous membrane include polyesters, polyamides, polyolefins, polysulfones, and celluloses, for example, which are already mentioned above. The second layer 53b is preferably formed of an inherently hydrophilic material or a material which has undergone the treatment intended to impart hydrophilicity. The methods for the impartation of hydrophilicity are the same as those already cited above.

The second layer 53b constructed as described above has the function to separate red blood cells as filtrate from the sample. To be specific, the porous membrane which forms the second layer 53b preferably contains pores of a diameter such that at least the red blood cells contained in the blood sample are filtered out by the membrane. Therefore, the diameter of the pores in the second layer 53b is preferably not more than 5 µm, more preferably in the approximate range of 0.2 - 3.5 µm. The second layer may be either an isotropic membrane or an anisotropic membrane. If the pore diameter is unduly large, the red blood cells will pass the second layer 53b and transfer to the basal face (measuring face) side of the test paper 53 and will possibly degrade the accuracy of measurement of the color which developed.

The first layer 53a and the second layer 53b may be joined throughout or partly in their entire surfaces or may be simply superposed without using any binding force.

The thickness of the second layer 53b, though not restricted particularly, is preferably in the approximate range of 10 - 1000 µm, more preferably in the approximate range of 50 - 200 µm. It should be opaque enough that the presence of filtrate such as red cells on its rear face will not affect readings from its test face.

A fixing part 533 is formed on the outer peripheral part of the test paper 53.

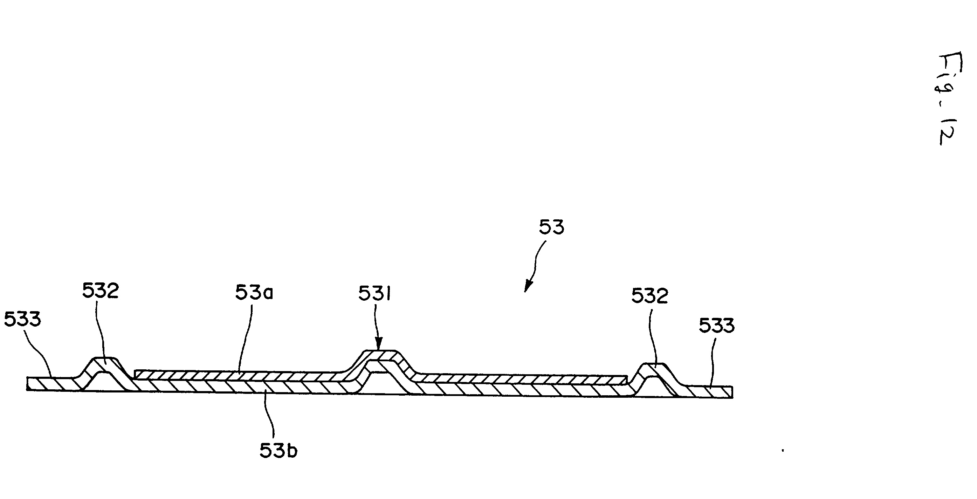

A plurality of fixing points 534 are formed intermittently (preferably as spaced at intervals of an equal distance) along the outer peripheral part of the test paper 53 as illustrated in Fig. 11. As a result, ambient air is allowed to pass through the intervals between the adjacent fixing points 534. When the blood flowing out of the sample outlet mouth 527 spreads on the test paper 53, the air entrapped in the gap 54 and the sample reservoir 55 is efficiently discharged and the spread of blood can be carried out more quickly.

The test paper 53 may be secured to the sample outlet side terminal part 525 by fusion or adhesion with adhesive agent. As a result, it is more stably supported and fixed on the cap 51 and possible deformations (bend, warp, wave, etc.) of the test paper 53 can be prevented; these could give rise to gaps, and obstruct the development of blood.

Fig. 13 is a side view illustrating the way in which the tip 5 is used for collecting blood. As illustrated therein, the collection of blood is begun by puncturing the finger tip (or the earlobe) with a needle or a surgical knife and forcing blood 18 to flow out through the puncture in a small amount (for example, in the approximate range of 2 - 6 µl) onto the skin.

Meanwhile, the tip 5 is loaded in the light measuring part 4 of the analyte measuring device 1 and the terminal face of the sample inlet side terminal part 521 of the tube 52 is brought into contact with the skin. The blood 18 on the finger tip flows through the groove 522 and reaches the sample inlet mouth 523 and, on being aspirated by capillarity, flows inside the sample inlet conduit 520 in the direction of the basal terminal and reaches the sample outlet mouth 527. At this time, there is no possibility of the blood 18 on the finger tip being excessively dispersed on the skin and wasted, since it is effectively aspirated through the lateral side opening part of the groove 522 (the part opened in the outer peripheral face of the tube 52).

The blood which has reached the sample outlet mouth 527 is brought into contact with and absorbed by the convex part 531 of the test paper 53 and part of the blood is allowed to pass through the groove 526 and reach the gap 54. The blood which has flowed into the gap 54 is absorbed and developed by the adjoining first layer 53a of the test paper 53 and meanwhile expanded radially in the direction of the outer periphery. As the absorption and development of the blood by the first layer 53a and particularly the absorption in the vicinity of the convex part 531 proceed, absorbing power continues to be effective in the sample inlet conduit 520 to cause a continuous supply of the blood to the first layer 53a.

Even when the amount of the blood 18 on the finger tip is comparatively small, the blood can be supplied to the first layer 53a (test paper 53) without any waste. Conversely, when the amount of the blood 18 on the finger tip is large and is supplied excessively to the first layer 53a, the possibility of the blood leading out of the test paper 53 and adhering to and smearing the inner face of the barrel part 52, the light measuring part 4, or their peripheral parts is precluded because the excess blood is retained in the sample reservoir 55 and prevented by the annular convex part 532 from flowing out toward the external periphery. The excess blood, therefore, is handled more safely because it exerts no adverse effect on the next round of measurement and has no possibility of causing infection during the disposal of the used tip 5.

In consequence of the supply and development of blood on the first layer 53a, the target analyte (glucose, for example) in the blood reacts with the reagent deposited on the first layer 53a and the test paper 53 assumes a color proportionately to the amount of the analyte. The blood components (excluding red blood cells) which have been colored migrate to the second layer 53b and color the second layer 53b in the same color tone with the same intensity. At this time, since the red blood cells in the blood are passed through the first layer 53a but are not passed through the second layer 53b, they stagnate in the vicinity of the boundary between the first layer 53a and the second layer 53b.

The target analyte (blood sugar level) in the blood is found by measuring the intensity of coloration of the second layer 53b with the analyte measuring device 1 in the manner described above. The red blood cells which are contained in the blood exert no adverse effect on the measurement of color by the test paper because they are filtered out by the second layer 53b, retained in the vicinity of the boundary between the first layer 53a and the second layer 53b, and prevented from migrating toward the basal terminal face of the second layer 53b. Thus, the accuracy of measurement can be high.

The harmful effect of red blood cells and the like is eliminated to ensure accuracy of measurement. Because the first layer 53a passes red blood cells and meanwhile develops coloration; this development proceeds quickly and uniformly. Also because the blood components (excluding red blood cells and any other filtrate) which have been colored within the first layer 53a migrate to the second layer 53b and color that second layer, with the result that the intensity of the resultant coloration can be used for measurement. For these reasons, the time required for the measurement can be shortened.

When the cap 5 is used, the blood 18 which has flowed out onto the finger tip can be supplied and developed on the test paper 53 quickly and infallibly by a simple procedure without reference to its amount. As a result, the measurement error is extremely small.

Specific examples of embodiment will now be described below.

Working Example 1

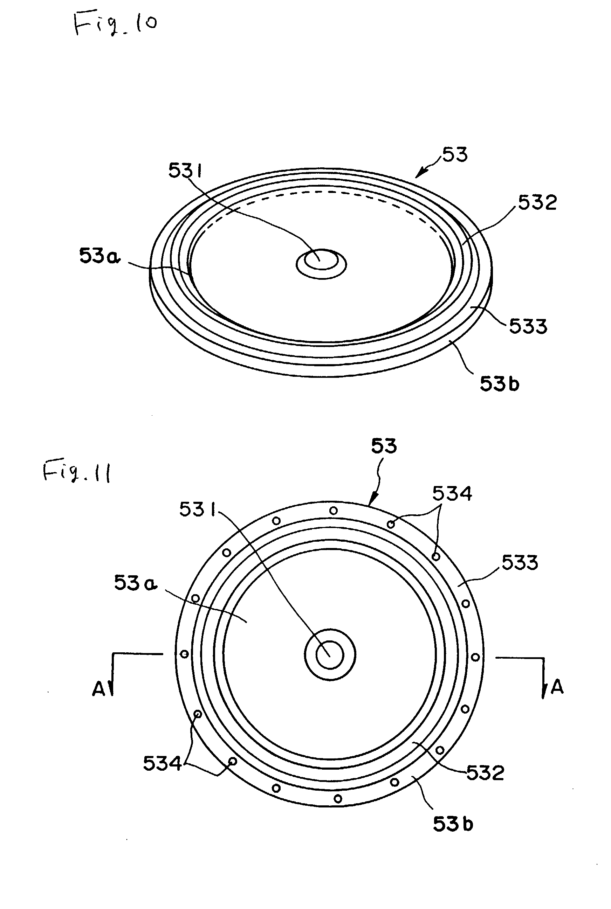

A blood sugar level measuring test paper was manufactured in the shape and construction (two-layer laminate) illustrated in Fig. 10 - Fig. 12. The conditions of the component parts of the test paper were as follows.

First layer 53a

Material: Nitrocellulose (hydrophilic) (made by Millipore Company)

　　Outside diameter: 4.2 mm
　　Thickness: 135 µm
　　Diameter of pores: 12 µm
　　Reagents deposited: GOD, POD, and 4-aminoantipyrine, N-ethyl-N-(2-hydroxy-3-sulfopropyl) -m-toluidine (TOOS)

Amounts of reagents deposited:

　　GOD - 250 µg/cm$^2$ test paper
　　POD - 125 µg/cm$^2$ test paper
　　4-Aminoantipyrine - 60 µg/cm$^2$ test paper
　　TOOS - 90 µg/cm$^2$ test paper

Second layer 53b

Material: Polyether sulfone (hydrophilic) (made by Pall Gelman Company)

Outside diameter: 5.4 mm
Thickness: 135 µm
Diameter of pores: 0.45 µm

Others

Diameter of convex part 531: 0.4 mm
Height of convex part 531: 0.3 mm
Inside diameter of annular convex part 532: 0.3 mm
Width of annular convex part 532: 0.3 mm
Height of annular convex part 532: 0.3 mm

This test paper was set in place in a tip of the construction illustrated in Fig. 4 - Fig. 8. The conditions of the component parts of this tip were as shown below.

Material of tip proper: Acryl resin (hydrophilic)
Outside diameter of tip proper: 9.5 - 11.0 mm
Inside diameter of tip proper: 9.0 - 10.0 mm
Inside diameter of sample inlet conduit, d1: 0.7 mm
Inside diameter of sample inlet conduit, d2: 0.7 mm
Length of sample inlet conduit: 4.2 mm
Peripheral length, L1: 0.7 mm (= Πd1 x 32%)
Depth of groove 522, P1: 0.5 mm
Peripheral length, L2: 0.7 mm (= Πd2 x 32%)
Depth of groove 526, P2: 0.3 mm
Width (depth) of gap 54: 0.1 mm
Depth of sample reservoir 55: 0.3 mm
Height of spacer 56: 0.5 mm
Method of fixing test paper: Fixed by fusion at the fixing points laid out as illustrated in Fig. 11.

Comparative Example

A blood sugar level measuring test paper formed of one layer of porous membrane was manufactured. The conditions of the component parts of the test paper were as shown below.

Porous membrane

Material: Polyether sulfone (hydrophilic) (made by Pall Gelman Company)

Outside diameter: 5.4 mm
Thickness: 135 µm
Diameter of pores: 0.45 µm
Reagent deposited and amount thereof: Identical to those of the working example described above.

Others

Diameter of convex part 531: Same as in the working example described above.
Height of convex part 531: Same as in the working example described above.
Inside diameter of annular convex part 532: Same as in the working example described above.
Width of annular convex part 532: Same as in the working example described above.
Height of annular convex part 532: Same as in the working example described above.
This test paper was set in place in the tip having the same conditions as in the working example described above.

Experiment 1

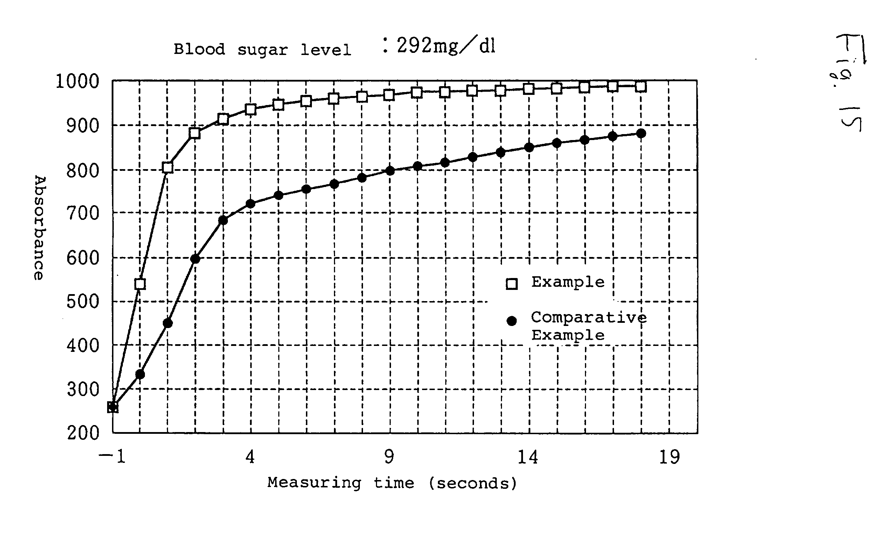

The following experiment was performed by using the tips of the working example and the comparative example.
As the sample, blood adjusted to have a blood sugar level of 186 mg/dl was prepared. Four (4) µl of this blood was brought into contact with the leading terminal of the slender tube of the tip and caused to be aspirated into the sample inlet conduit. The time at which the blood supplied to the convex part 531 at the center of the test paper began to be diffused radially from the convex part 531 was taken as the measuring time, 0 (second). At intervals of one second between the first through 18th second, the light projected on the blood supply side of the test paper and received on the opposite side were repeated for the measurement of the absorbance of the reflected light. The results are represented by the graph of Fig. 14. The absorbance in the graph was computed in accordance with the following formula (III).

$$\text{Absorbance} = [\text{Intensity of reflected light (before development of blood)}]/[\text{Intensity of reflected light (after development of blood)}] \times 256 \quad \text{(III)}$$

Experiment 2

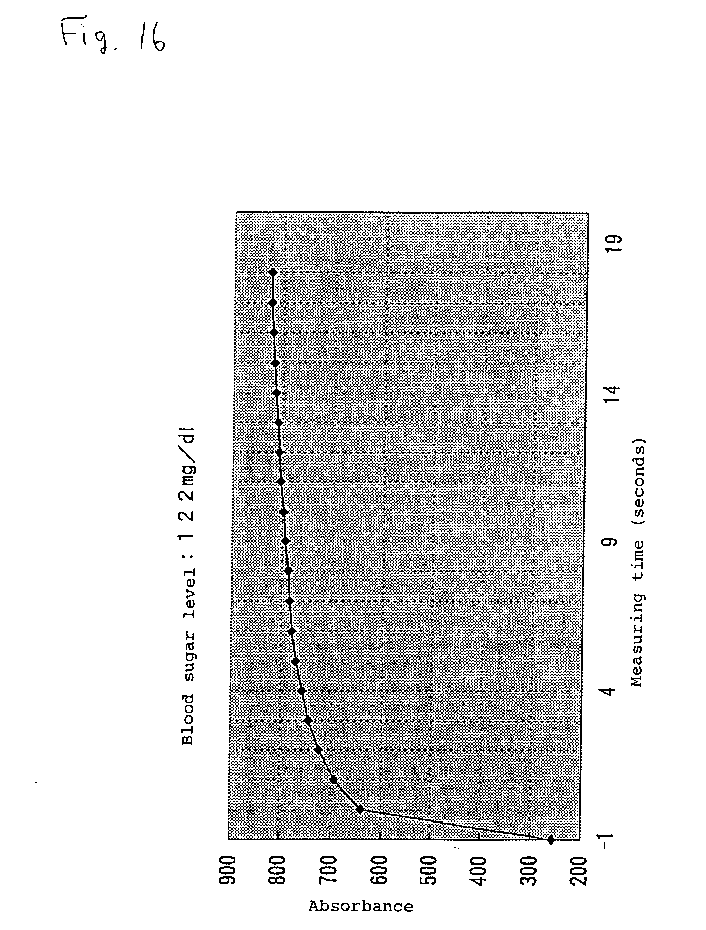

An experiment was performed by following the procedure of the experiment 1 described above while using blood adjusted to have a blood sugar level of 292 mg/dl as the sample instead. The results are represented by the graph in Fig. 15. The absorbance in the graph was computed in accordance with the formula (III) mentioned above.
It is clearly noted from the graphs of Fig. 14 and Fig. 15 that the working example of this invention, as compared with the comparative examples, developed the sample at a high speed and the intensity of coloration (absorbance of reflected light) was stabilized within a brief time. By using the time at which the graph substantially reached equilibrium as the end point of the measurement, the blood sugar level could be measured quickly with high accuracy.

Working Example 2

A test paper was produced by following the procedure of Working Example 1 while using

a first layer of a polyester (non-woven fabric) (made by Tonen K.K.) having a pore diameter of 16 μ and a second layer of an anisotropic polyether sulfone (made by Fuji Photo Film Co., Ltd.) having a pore diameter of 0.45 μ and this test paper was loaded on a chip illustrated in Fig. 4 - Fig. 8.

Working Example 3

A test paper was produced by following the procedure of Working Example 1 while using

a first layer of a nitrated polyether sulfone (made by Pall Gelman Corp) and
a second layer of an anisotropic polyether sulfone (made by Fuji Photo Film Co., Ltd.) having a pore diameter of 0.45 μ and this test paper was loaded on a chip illustrated in Fig. 4 - Fig. 8.

Experiment 3

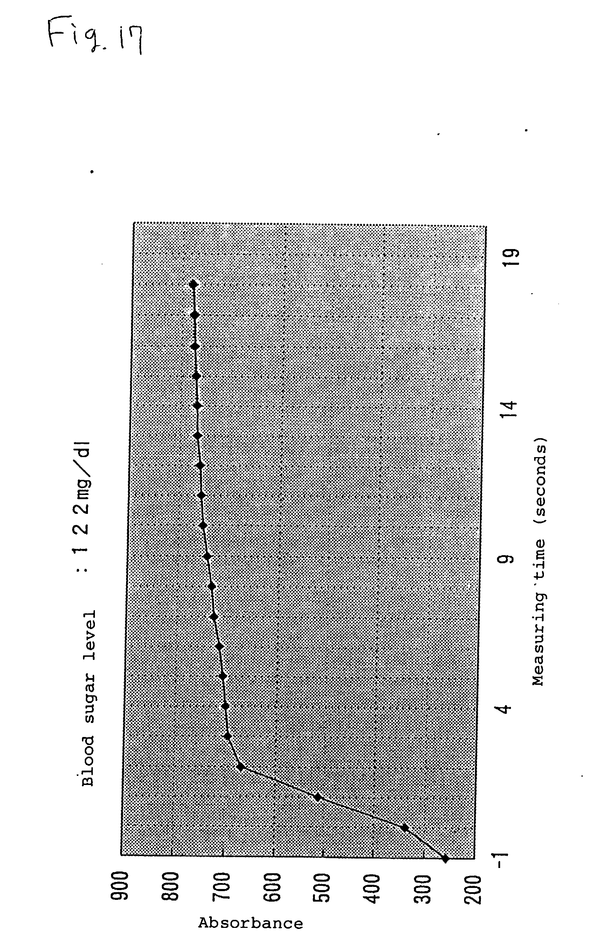

An experiment was performed on the chip of Working Example 2 by following the procedure of Experiment 1 while using blood adjusted to have a blood sugar level of 122 mg/dl as the sample instead. The results are represented by the graph in Fig. 16.

Experiment 4

An experiment was performed on the chip of Working example 3 by following the procedure of Experiment 3. The results are shown by the graph of Fig. 17.
While there have been shown and described the test paper and the analyte measuring tip of this invention with reference to the accompanied drawings, it is to be distinctly understood that the invention is not limited thereto.
The test paper of this invention does not need to be limited to what is loaded on the tip 5 as described above. The test paper may be manufactured arbitrarily in various forms such as, for example, what is formed solely of itself, what is superposed on a basis (substrate) shaped like a plate or sheet, or what is retained (particularly nipped) by a holder.
The working example, as described above, used blood as the sample. This invention does not need to limit the sample to blood. The sample may be urine, lymph, cerebrospinal fluid, saliva, or other similar bodily humor, or a diluted solution thereof, or a concentrated solution thereof.
The analyte aimed at by the measurement does not need to be limited to grape sugar (blood sugar level). For example, it may be protein, cholesterol, uric acid, creatine, alcohol, sodium or other inorganic ion, or hemoglobin (occult blood).
The analyte measuring device fitted with the test paper or the analyte measuring tip according to this invention does not need to be limited to what operates by optically measuring the intensity of color of the test paper which has assumed the color in consequence of the reaction of the analyte in a sample with the reagent, computing the magnitude of measurement based on the result of the optical measurement, and displaying the outcome of the computation. It may be adapted to operate by electrically measuring a potential change which arises proportionately to the amount of the analyte in the sample, computing the magnitude of measurement based on the outcome of the electrical measurement, and displaying the magnitude.
As described above, the test paper and the analyte measuring tip according to this invention can heighten the speed of development of the sample on the test paper and curtail the time required for the development. They also shortens the time which precedes the stabilization of the state of coloration. As a result, the measurement can be expedited. The measurement of color can be attained with high accuracy by eliminating the otherwise possible effect of red blood cells and the like.
Further, the analyte measuring tip according to this invention allows convenient, quick, and infallible collection of a sample and effects necessary development of the sample on the test paper without reference to the conditions of the sample such as, for example, constitution and amount of the sample, site of collection, and technique of collection. As a result, it is capable of affording an accurate magnitude of measurement.
The analyte measuring tip allows easy handling and simplifies the operation of attachment thereof to the analyte measuring device. Particularly, it can be easily and infallibly loaded in a proper state. The possibility of involving error of measurement due to improper state of attachment, therefore, can be precluded.
The contamination due to adhesion of the sample can be prevented and the used analyte measuring tip can be discarded with high safety.
Owing to the factors enumerated above, the analyte measuring device contributes advantageously to the automation of the measurement.

**Claims**

1. A test paper (53) for colorimetric assessment of an analyte at a test face thereof characterized by having a first porous layer (53a) having therein a reagent capable of assuming a color on reacting with an analyte in a sample and a second porous layer (53b) for separating a filtrate from said sample emanating from the first layer, the test face being a face of the second layer remote from the first layer.

2. A test paper according to claim 1, wherein said first layer and said second layer are each hydrophilic.

3. A test paper according to claim 1 or claim 2, wherein pores in said first layer have diameters in the range of 8 - 50 μm.

4. A test paper according to any of claims 1 to 3, wherein pores in said second layer have diameters of not more than 5 μm.

5. A test paper according to any of claims 1 to 4, wherein said sample is to be blood and the paper is adapted to separate as said filtrate blood cells which are primarily red blood cells.

6. An analyte measuring method using a measuring tip (5) on an analyte measuring device (1) the tip (5) being provided with a tube (52) projecting from said tip to form a conduit for introducing a sample to the inside of the tip, and a test paper (53) according to any of claims 1 to 5 disposed inside the tip, the method including allowing a sample supplied to an inlet (523) of said tube (52) to be conveyed by virtue of capillarity of said tube to an outlet (52) thereof, supplying and developing said sample toward said first layer (53a) of said test paper, filtering the sample in the second layer (53b) and measuring the color of the sample at said test face side of second layer (53b).

7. A tip (5) provided at one end portion thereof with a loading part for fitting to an analyte measuring device (1) characterised in that it has an oppositely-projecting tube (52) forming a sample conduit (520) intended to collect a sample and in that it has disposed in communication with the sample outlet (527) of said tube a test paper (53) according to any of claims 1 to 5, for capillary delivery of the sample along the conduit (520) to the test paper (53).

8. A tip according to claim 7, wherein said tube (52) has in the sample inlet (523) thereof a first groove (522) communicating with said sample conduit (520).

9. A tip according to claim 8, wherein said first groove (522) extends diametrically of said tube (52) and opens on the outer peripheral surface of said tube (52).

10. A tip according to any of claims 7 to 9, wherein said tube (52) has at the sample outlet (527) thereof a second groove (526) communicating with said sample conduit (520).

11. A tip according to claim 10, wherein a part (525) projects inwardly adjacent said sample outlet (527) and said second groove (526) is formed in said projecting part to extend diametrically of said tube and opens to an outer peripheral surface of said projecting part (525).

12. A tip according to any of claims 7 to 11, wherein the test paper has confronting said sample conduit (520) a convex part (531) protruding toward said sample conduit.

13. A tip according to claim 12, wherein at least the leading portion of said convex part (531) is inserted into said sample conduit (520).

14. A tip according to any of claims 6 to 13, wherein the test paper has within its outer periphery an annular convex part (532).

15. A tip according to claim 14, wherein the first layer (53a) of said test paper terminates within the radially inner periphery of said annular convex part (532).

16. A tip according to any of claims 6 to 15, wherein said test paper is provided on the outer peripheral portion thereof with a part (533) fixed to an inner face (512) of said tip.

17. A tip according to claim 16, wherein said part (533) is fixed intermittently to the tip by fusion or adhesion.

18. A tip according to any of claims 6 to 17, which is loaded on a test paper loading part (43) of said analyte measuring device (1) and is provided with separating means (56) for ensuring absence of contact between said test paper (53) and said test paper loading part (43) during the loading.

19. A tip according to claim 18, wherein said separating means is a spacer (56) projecting on the inner face of said tip and adapted to collide against said test paper loading part (43).

20. A tip according to any of claims 7 to 19, wherein the inner diameter of said sample inlet conduit is in the range 0.3 - 1.0 mm.

21. A tip according to any of claims 7 to 20, wherein the length of said sample inlet conduit is in the range 2 - 5 mm.

Fig. 1

Fig. 2

Fig. 3

```
┌─────────────────────┐                                          ┌──────────────────────┐
│ Liquid crystal      │──9                                  10   │ Control              │──11
│ display device      │◄──────┐        ┌──────────────┐    ┌────►│ oscillating part     │
└─────────────────────┘       │        │              │    │     └──────────────────────┘
                              │        │              │◄───┘
┌─────────────────────┐──6    │        │              │          ┌──────────────────────┐
│ Power source        │       │        │              │◄─────────│ Clock                │
│ part                │───────┼───────►│              │          │ oscillating part     │
└─────────────────────┘       │        │              │          └──────────────────────┘
         │                    │        │   Control    │                              12
         ▼                    │        │    means     │          ┌──────────────────────┐──13
┌─────────────────────┐──7    │        │              │◄────────►│ Data memory part     │
│ Power source        │       │        │              │          └──────────────────────┘
│ voltage detecting   │───────┼───────►│              │
│ part                │       │        │   (M P U)    │          ┌──────────────────────┐──14
└─────────────────────┘       │        │              │─────────►│ Buzzer output part   │
                              │        │              │          └──────────────────────┘
┌─────────────────────┐──8    │        │              │
│                     │       │        │              │          ┌──────────────────────┐──15
│ Switch circuit      │───────┼───────►│              │─────────►│ External output      │
│                     │       │        │              │          │ part                 │
└─────────────────────┘       │        │              │          └──────────────────────┘
                              │        │              │
┌─ ─ ─ ─ ─ ─ ─ ─ ─ ─ ─┐──41  │        │              │          ┌──────────────────────┐
│ ┌─────────────────┐ │      │        │              │◄─────────│ Temperature          │
│ │ Luminescent     │◄┼──────┘        │              │          │ measuring part       │
│ │ element         │ │               └──────────────┘          └──────────────────────┘
│ └─────────────────┘ │                   ▲   ▲                                      16
│ ┌─────────────────┐ │                   │   │
│ │ Light-receiving │ │               ┌───┴───┴──────┐
│ │ element         │─┼──────────────►│  A/D         │
│ └─────────────────┘ │               │  converter   │
└─ ─ ─ ─ ─ ─ ─┬─ ─ ┬─ ┘               └──────────────┘
              4    42                       44
```

Fig. 4

Fig. 5

Fig. 6

Fig. 7

Fig. 8

Fig. 9

Fig. 10

Fig. 11

Fig. 12

Fig. 13

Fig. 14

EP 0 926 484 A2

Fig. 15

Blood sugar level ：292mg／dl

EP 0 926 484 A2

Fig. 16

Blood sugar level : 1 2 2mg/dl

Measuring time (seconds)

EP 0 926 484 A2

Fig.17

Blood sugar level  : 1 2 2mg/dl

Absorbance

Measuring time (seconds)